# EUROPEAN PATENT SPECIFICATION

(11) **EP 2 210 950 B1**
(45) Date of publication and mention of the grant of the patent: **14.08.2013**
(21) Application number: 09168440.7
(22) Date of filing: 24.08.2009
(51) Int. Cl.: C12N 15/70

(54) **Plasmid for expressing thioredoxin fusion protein and method for producing target protein using same**
Plasmid zur Expression eines Thioredoxin-Fusionproteins und Verfahren zur Herstellung des Zielproteins damit
Plasmide pour l'expression de la protéine de fusion thiorédoxine et procédé pour la fabrication de protéine cible l'utilisant

(30) Priority: 15.01.2009 KR 20090003431
(43) Date of publication of application: 28.07.2010
(73) Proprietor: Korea Institute of Science and Technology, Seoul 136-791 (KR)
(72) Inventor: Ahn, Hee-Chul, 135-834 Seoul (KR); Ahn, Hyung Jun, 130-010 Seoul (KR); Shin, Dong Yun, 135-110 Seoul (KR); Lim, Jong Soo, 156-846 Seoul (KR); Ahn, Dae-Ro, Gyeonggi-do 413-735 (KR)
(74) Representative: Grünberg, Thomas

(56) References cited:
- WO-A1-92/13955
- PEISLEY ET AL: "High-level expression of a soluble and functional fibronectin type II domain from MMP-2 in the Escherichia coli cytoplasm for solution NMR studies" PROTEIN EXPRESSION AND PURIFICATION, ACADEMIC PRESS, SAN DIEGO, CA LNKD- DOI:10.1016/J.PEP.2006.12.005, vol. 53, no. 1, 24 February 2007 (2007-02-24), pages 124-131, XP005899732 ISSN: 1046-5928
- TREY SIMMONS ET AL: "Human Low Density Lipoprotein Receptor Fragment. SUCCESSFUL REFOLDING OF A FUNCTIONALLY ACTIVE LIGAND-BINDING DOMAIN PRODUCED IN ESCHERICHIA COLI" JOURNAL OF BIOLOGICAL CHEMISTRY, AMERICAN SOCIETY FOR BIOCHEMISTRY AND MOLECULAR BIOLOGY, INC, US, vol. 272, no. 41, 10 October 1997 (1997-10-10), pages 25531-25536, XP007912702 ISSN: 0021-9258
- DELUCCHI A B ET AL: "Synthesis of <32>P-labelled protein probes using a modified thioredoxin fusion protein expression system in Escherichia coli" BIOMOLECULAR ENGINEERING, ELSEVIER, NEW YORK, NY, US LNKD- DOI:10.1016/S1389-0344(02)00050-3, vol. 20, no. 1, 1 January 2003 (2003-01-01), pages 1-5, XP004399384 ISSN: 1389-0344
- LAVALLIE E R ET AL: "A THIOREDOXIN GENE FUSION EXPRESSION SYSTEM THAT CIRCUMVENTS INCLUSION BODY FORMATION IN THE E. COLI CYTOPLASM" BIO/TECHNOLOGY, NATURE PUBLISHING CO. NEW YORK, US LNKD- DOI:10.1038/NBT0293-187, vol. 11, no. 2, 1 February 1993 (1993-02-01), pages 187-193, XP000195207 ISSN: 0733-222X
- NOVAGEN: "pET-32a-c(+) Vectors" INTERNET CITATION December 1998 (1998-12), pages 1-2, XP007912744 Retrieved from the Internet: URL:https://wasatch.biochem.utah.edu/chris /links/pET32.pdf> [retrieved on 2010-04-21]

## Description

### FIELD OF THE INVENTION

The present invention relates to a plasmid for expressing a thioredoxin fusion protein, an *Escherichia coli* cell transformed with the plasmid, and a method for producing a target protein using the *E. coli* cell.

### BACKGROUND OF THE INVENTION

A variety of fusion partners, e.g., glutathione-S-transferase (Smith, D. B. et al., Gene, 67: 31-40, 1988), maltose-binding protein (Bedouelle, H. et al., Eur. J. Biochem., 171: 541-549, 1988), protein A (Nilsson, B. et al., Mol. Recog., 1: 69-74, 1988) and thioredoxin (Navallie, E. R. et al., Biotechnol., 11: 187-193, 1993), have been widely used for expressing soluble forms of biologically active heterologous proteins in *E. coli* cells. T hioredoxin, one of the most useful fusion partners, is a protein found in nearly all organisms, which reduces disulfide bonds in proteins into thiols.

Examples of plasmids for expressing thioredoxin fusion proteins include p-BADM-20 (EMBL-Hamburg) having araB promoter site and pET-32 (Novagen) having T7 promoter site, the use of pET-32 plasmid being generally preferred in the art.

The pET-32 plasmid expresses a thioredoxin fusion protein containing an S-tag protein. To selectively obtain a target protein, the thioredoxin fusion protein may be digested with enterokinase, whose cost is, however, high. When such a thioredoxin fusion protein is digested with less expensive thrombin, the target protein may still have the S-tag protein attached thereto, which complicates the structural determination of the target protein. To circumvent this problem, there has been developed a method of placing a gene encoding a protease recognition site in the front of a heterologous protein gene, which comprises inserting a gene encoding protease recognition site in a primer used for PCR amplification of the h eterologous protein gene. In this method, the protease recognition site is inserted between the amino acid sequence of the heterologous protein and S-tag coding sequence, to make it possible to separate thioredoxin and S-tag proteins simultaneously using a suitable protease (e.g., TEV protease) for the purpose of obtaining only the target proteins. However, the method is cumbersome in that a protease recognition site must be inserted in a primer every time when a specific protein is to be expressed.

Taking note of the fact that another cleavage enzyme recognition site (i.e., thrombin recognition site) is present in pET-32a plasmid, the present inventors have constructed a novel plasmid from the pET-32a plasmid by deleting S-tag coding sequence via site-specific deletion mutagenesis, and have found that thioredoxin can be easily and selectively generated by treating a fusion protein expressed by said plasmid with thrombin.

### SUMMARY OF THE INVENTION

Accordingly, it is an object of the present invention to provide a plasmid for expressing thioredoxin fusion proteins from which thioredoxin can be easily removed.

It is another object of the present invention to provide a plasmid wherein a gene encoding a target protein is inserted in the multiple cloning site of the plasmid described above.

It is a further object of the present invention to provide an *E.coli* cell transformed with said plasmids.

It is a still further object of the present invention to provide a method for producing a target protein using the transformed *E.coli* cell.

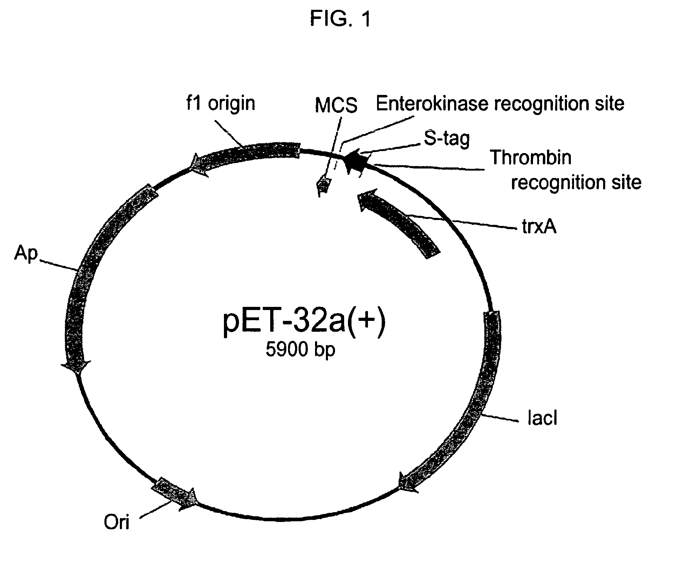

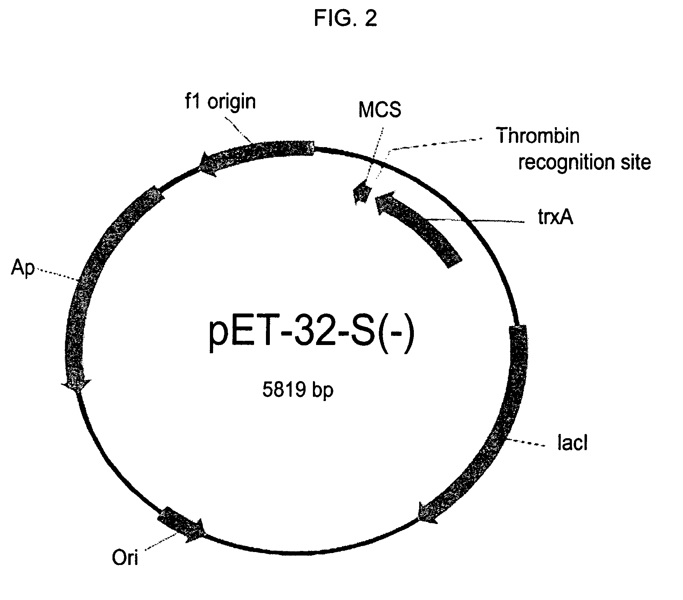

In accordance with one aspect of the present invention, there is provided a plasmid which is prepared by deleting the S-tag coding sequence from pET-32a(+) plasmid having the nucleotide sequence of SEQ ID NO:3 represented by the cleavage map of Fig. 1, the pET-32a(+) plasmid comprising a thioredoxin gene, a thrombin recognition site, an S-tag coding sequence, and a multiple cloning site for the insertion of a gene encoding a target protein. Further, there is provided the plasmid having the nucleotide sequence of SEQ ID NO: 4 represented by the cleavage map of Fig.2, and is designated pET-32-S(-) plasmid.

In accordance with another aspect of the present invention, there is provided the plasmid wherein the gene encoding a target protein is inserted in the multiple cloning site of the pET-32-S(-) plasmid.

In accordance with a further aspect of the present invention, there is provided an *E.coli* cell transformed with the plasmid.

In accordance with a still further aspect of the present invention, there is provided a method for producing a target protein, comprising i) inserting a gene encoding the target protein in the multiple cloning site of pET-32-S(-) plasmid; ii) transforming *E.coli* cells with the plasmid obtained in i), and culturing the transformed *E.coli* cells to express a thioredoxin fusion protein; and iii) treating the thioredoxin fusion protein with thrombin.

### BRIEF DESCRIPTION OF THE DRAWINGS

The above and other objects and features of the present invention will become apparent from the following description of the invention, when taken in conjunction with the accompanying drawings, which respectively show:
Fig. 1: the cleavage map of pET-32a(+) plasmid available from Novagen (MCS: multiple cloning site, trxA: thioredoxin gene, lacI: lac repressor coding gene, Ori: origin for replication, Ap: ampicillin-resistance gene);
Fig. 2: the cleavage map of pET-32-S(-) plasmid prepared by deleting the S-tag sequence from pET-32a(+) plasmid (MCS: multiple cloning site, trxA: thioredoxin gene, lacI: lac repressor coding gene, Ori: origin for replication, Ap: ampicillin-resistance gene);
Fig. 3: a diagram showing bonding pairs between primers used for deleting the S-tag sequence of pET-32a(+) plasmid via site-specific deletion mutagenesis and DNAs of pET-32a(+) plasmid (MCS: multiple cloning site, trxA: thioredoxin gene, lacI: lac repressor coding gene, Ori: origin for replication, Ap: ampicillin-resistance gene); and
Fig. 4: SDS-PAGE analysis results showing the removal of thrombin from a thioredoxin-fused STAT6 SH2 protein which is produced by inserting STAT6 SH2 protein coding sequence into the pET-32-S(-) plasmid and transforming *E*. *coli* cells with the plasmid.

### DETAILED DESCRIPTION OF THE INVENTION

Hereinafter, the present invention is described in detail.

The plasmid of the present invention, which over-expresses a target protein using thioredoxin as a fusion partner, is prepared by deleting the S-tag coding sequence from pET-32a(+) plasmid having the nucleotide sequence of SEQ ID NO: 3 represented by the cleavage map of Fig. 1, the pET-32a(+) plasmid comprising a thioredoxin gene, a thrombin recognition site, an S-tag coding sequence, and a multiple cloning site for the insertion of a gene encoding a target protein. The plasmid of the present invention has the nucleotide sequence of SEQ ID NO: 4 represented by the cleavage map of Fig. 2 and is designated pET-32-S(-) plasmid.

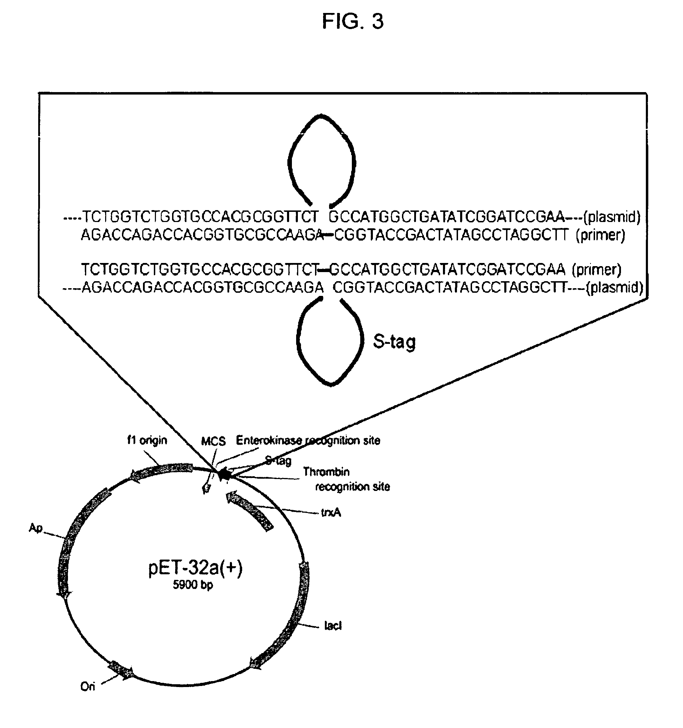

Specifically, the pET-32-S(-) plasmid is characterized by that a thrombin recognition site is inserted between the thioredoxin gene and multiple cloning site, and that the S-tag coding sequence does not exist. The pET-32-S(-) plasmid is prepared by deleting the S-tag coding sequence from the pET-32a(+) plasmid (Novagen; SEQ ID NO: 3) represented by the cleavage map of Fig. 1, through the induction of site-specific deletion mutagenesis via polymerase chain reaction(PCR) using primer pairs of SEQ ID NO: 1 and SEQ ID NO: 2 as shown in Fig 3.

The plasmid of the present invention comprises T7 promoter for controlling the transcription and expression of a fusion protein as pET-32a(+) plasmid does. In the plasmid of the present invention, the S-tag sequence does not exist and the thrombin recognition site is located in front of the multiple cloning site (5' side) for the purpose of obtaining only the target protein easily and selectively after the isolation and purification of a fusion protein.

The present invention also provides the plasmid wherein the gene encoding a target protein is inserted in the multiple cloning site of said pET-32-S(-) plasmid.

Said target protein is an active protein which can be easily expressed in *E*. *coli* in a soluble form when fused with thioredoxin. The target protein may be selected from the group consisting of human STAT6 SH2 domain, prolyl hydroxylase 2 (PHD2), human β-defensin-2 (hBD2), human interleukin-6 receptor α-chain (hIL-6Rα), mannan-binding lectin-CLR (MBL-CLR), wheat peroxisomal ascorbate peroxidase (pAPX), rhesus macaque interleukin-4(rMamu IL-4), and α-galactosidase.

The plasmid of the present invention may comprise a histidine tag (His-tag) (e.g. six histidine tag), a polyarginine or a consensus biotinylated peptide coding sequence to be linked in the form of translational fusion, at amino terminus and/or carboxyl terminus. Accordingly, the produced fusion protein can be easily isolated and purified using various affinity chromatographies such as nickel chelation column chromatography (e.g. Ni-NTA affinity chromatography).

The present invention also provides an *E*. *coli* cell transformed with the plasmid of the present invention. Examples of the *E*. *coli* cell include *E*. *coli* DH5α, BL21, HB101, JM109 and TH2 etc, but not limited thereto.

The present invention also provides a method for producing a target protein, comprising: i) inserting a gene encoding the target protein in the multiple cloning site of the pET-32-S(-) plasmid; ii) transforming *E*. *coli* cells with the plasmid obtained in i), and culturing the transformed *E*. *coli* cells to express a thioredoxin fusion protein; and iii) treating the thioredoxin fusion protein with thrombin.

In the method of the present invention, the target protein is an active protein which can be easily expressed in *E*. *coli* in a soluble form when fused with thioredoxin, and may be selected from the group consisting of human STAT6 SH2 domain, prolyl hydroxylase 2 (PHD2), human β-defensin-2 (hBD2), human interleukin-6 receptor α-chain (hIL-6Rα), mannan-binding lectin-CLR (MBL-CLR), wheat peroxisomal ascorbate peroxidase (pAPX), rhesus macaque interleukin-4(rMamu IL-4), and α-galactosidase.

The method of the present invention may further comprise steps of isolation and purification of the thioredoxin fusion protein, prior to step (iii). The isolation and purification steps may be carried out using an affinity column chromatography such as nickel chelation column chromatography.

The following Examples are intended to further illustrate the present invention without limiting its scope.

### Example 1: Construction of pET-32-S(-) plasmid by site-specific deletion mutagenesis of pET-32a(+) plasmid.

Using a PCR solution composed of pET-32a(+) plasmid of SEQ ID NO: 3 (Novagen) as a template (0.5 µL, 50 ng/µL); primers of SEQ ID NOs: 1 and 2 (in concentration of 1 µL, 10 µL); dNTP mix (1 µl, 100 mM); H₂O (40 µL); and pfu ultra-polymerase (1 µL), PCR was performed under the following conditions: predenaturation at 95°C for 5 min; denaturation at 95°C for 30 sec, annealing at 55°C for 1 min, followed by 20 cycles of polymerization at 68°C for 7 min; and final extension at 68°C for 7 min. The resulting PCR product was digested with restriction enzyme *Dpn* I at 37°C for 1 hour. Then, HIT^{™} competent DH5α cells (Real Biotech) was transformed with the above PCR product, and plasmid DNA was purified therefrom. The sequence analysis of the isolated DNA showed the polynucleotide sequence of SEQ ID NO: 4 (see the structure of Fig. 2), which lacks the S-tag sequence. This plasmid was designated as pET-32-S(-).

### Example 2: Cloning of a target gene into pET-32-S(-) and construction of a gene expression plasmid.

STAT6 proteins are phosphorylated by the receptor-associated kinases which are activated by cytokines or growth factors, and play a central role in signal transduction pathway. In order to obtain a sequence encoding SH domain (from 523^{th} to 661^{th} amino acids in STAT6 protein) of STAT6 proteins, using human STAT6 cDNA of SEQ ID NO: 5 (accession number: NM_003153; OriGene Technologies) as a template and primer pair of SEQ ID NOs: 6 and 7, PCR was performed under the following conditions: predenaturation at 95°C for 5 min; denaturation at 95°C for 30 sec, annealing at 58°C for 1 min, followed by 25 cycles of polymerization at 72°C for 1 min; and final extension at 72°C for 7 min.

The resulting PCR product was digested with restriction enzymes *Eco*RI and XhoI, and pET-32-S(-) plasmid obtained in Example 1 was treated with same enzymes. Then, the above PCR product and pET-32-S(-) plasmid were each isolated and purified by electrophoresis on 1% agarose gel, and were ligated each other using T4 DNA ligase at room temperature for 1 hour to obtain a plasmid construct. HIT^{™} competent DH5α cells (Real Biotech) were transformed with the plasmid construct, followed by isolating the plasmid DNA expressing STAT6 SH2 domain from the transformed cells.

### Example 3: Production and identification of a target protein.

*E.coli* BL21 (DE3) cells were transformed with the plasmid DNA isolated in Example 2. Then, the transformed colonies were inoculated in LB medium (5mL) containing 50 mg/mL of ampicillin, and cultured overnight at 37°C.

The resulting culture medium was added to 800 mL of LB medium, and expression of protein was induced by addition of IPTG (0.25 mM) when the cell density reached O.D=0.6 at 600 nm. Ce lls were harvested by centrifugation at 4000 rpm for 15 min using centrifugal separator (Sorvall), and were suspended in 30 mL of PBS buffer solution. Then, lysozyme (100mg/mL, 300 µL), 2-mercaptoethanol (30 µL) and phenylmethanesulfonylfluoride (PMSF) (100 mM, 150 µL) were added thereto, and the cells were sonicated and centrifuged at 13,000 rpm. From the obtained supernatant, thioredoxin fusion protein was isolated using Ni-NTA (Quiagen) resin. The obtained fusion protein was treated with thrombin, and cultured overnight at room temperature, to remove thioredoxin from the fusion protein. The obtained protein was isolated using fast protein liquid chromatography (FPLC), and was identified by 15% SDS-PAGE and Coomassie blue staining.

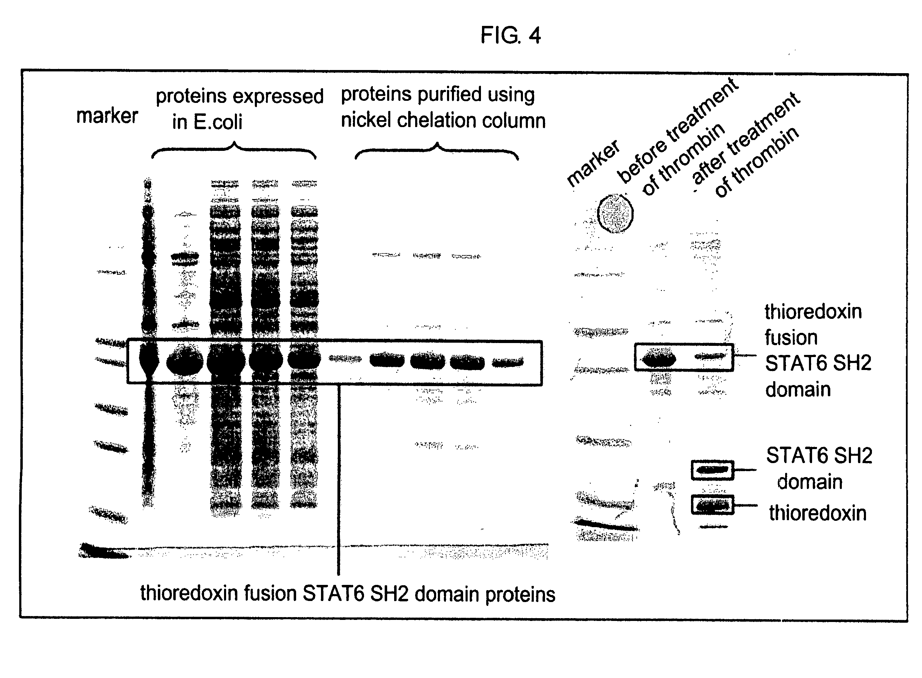

As shown in Fig. 4, it was confirmed that thioredoxin was easily removed by treating thrombin to a thioredoxin fusion protein, STAT6 SH2.

While the invention has been described with respect to the above specific embodiments, it should be recognized that various modifications and changes may be made to the invention by those skilled in the art which also fall within the scope of the invention as defined by the appended claims.
## Claims

1. A plasmid which is prepared by deleting the S-tag coding sequence from pET-32a(+) plasmid having the nucleotide sequence of SEQ ID NO: 3 represented by the cleavage map of Fig. 1, the pET-32a(+) plasmid comprising a thioredoxin gene, a thrombin recognition site, an S-tag coding sequence, and a multiple cloning site for the insertion of a gene encoding a target protein, wherein the plasmid has the nucleotide sequence of SEQ ID NO: 4 represented by the cleavage map of Fig.2 and is designated pET-32-S(-) plasmid.

2. The plasmid of claim 1, wherein the gene encoding a target protein is inserted in the multiple cloning site of the pET-32-S(-) plasmid.

3. The plasmid of claim 2, wherein said target protein is selected from the group consisting of human STAT6 SH2 domain, prolyl hydroxylase 2 (PHD2), human β-defensin-2 (hBD2), human interleukin-6 receptor α-chain (hIL-6Rα), mannan-binding lectin-CLR (MBL-CLR), wheat peroxisomal ascorbate peroxidase (pAPX), rhesus macaque interleukin-4 (rMamu IL-4), and α-galactosidase.

4. An *E*. *coli* cell transformed with the plasmid according to any one of claims 1 to 2.

5. The *E*. *coli* cell of claim 4, which is selected from the group consisting of DH5α, BL21, HB101, JM109, and TH2.

6. A method for producing a target protein, comprising:
i) inserting a gene encoding the target protein in the multiple cloning site of the plasmid of claim 1;
ii) transforming *E. coli* cells with the plasmid obtained in i), and culturing the transformed *E. coli* cells to express a thioredoxin fusion protein; and
iii) treating the thioredoxin fusion protein with thrombin.

7. The method of claim 6, wherein the target protein is selected from the group consisting of human STAT6 SH2 domain, prolyl hydroxylase 2(PHD2), human β-defensin-2 (hBD2), human interleukin-6 receptor α-chain (hIL-6Rα), mannan-binding lectin-CLR (MBL-CLR), wheat peroxisomal ascorbate peroxidase (pAPX), rhesus macaque interleukin-4 (rMamu IL-4), and α-galactosidase.

8. The method of claim 6, which further comprises steps of isolation and purification of the thioredoxin fusion protein prior to step (iii).

## Patentansprüche

1. Plasmid, welches durch Entfernen der das S-Tag kodierenden Sequenz aus dem die durch die Restriktionskarte in Fig. 1 dargestellte Nukleotidsequenz der SEQ ID NO: 3 aufweisenden pET-32a(+)-Plasmid hergestellt wird, wobei das pET-32a(+)-Plasmid ein Thioredoxin-Gen, eine Thrombin-Erkennungsstelle, eine S-Tag-Kodierungssequenz und eine multiple Klonierungsstelle für das Einfügen eines ein Targetprotein kodierenden Gens umfasst, wobei das Plasmid die durch die Restriktionskarte in Fig. 2 dargestellte Nukleotidsequenz der SEQ ID NO: 4 aufweist und als pET-32-S(-)-Plasmid bezeichnet ist.

2. Plasmid nach Anspruch 1, wobei das ein Targetprotein kodierende Gen in die multiple Klonierungsstelle des pET-32-S(-)-Plasmids eingefügt ist.

3. Plasmid nach Anspruch 2, wobei das Targetprotein aus der Gruppe bestehend aus humaner STAT6 SH2-Domäne, Prolyl-Hydroxylase 2 (PHD2), humanem β-Defensin-2 (hBD2), humaner Interleukin-6-Rezeptor-α-Kette (hIL-6Rα), Mannan bindendem Lectin-CLR (MBL-CLR), peroxisomaler Ascorbatperoxidase (pAPX) des Weizens, Rhesusaffen-Interleukin-4 (rMamu IL-4) und α-Galactosidase gewählt ist.

4. *E. coli* Zelle, die mit dem Plasmid nach einem der Ansprüche 1 und 2 transformiert ist.

5. *E. coli* Zelle nach Anspruch 4, die aus der Gruppe bestehend aus DH5α, BL21, HB101, JM109 und TH2 gewählt ist.

6. Verfahren zum Herstellen eines Targetproteins, umfassend:
i) Einfügen eines das Targetprotein kodierenden Gens in die multiple Klonierungsstelle des Plasmids nach Anspruch 1;
ii) Transformieren der *E*. *coli* Zellen mit dem in i) erhaltenen Plasmid, und Kultivieren der transformierten *E*. *coli* Zellen zum Exprimieren eines Thioredoxin-Fusionsproteins; und
iii) Behandeln des Thioredoxin-Fusionsproteins mit Thrombin.

7. Verfahren nach Anspruch 6, wobei das Targetprotein aus der Gruppe bestehend aus humaner STAT6 SH2-Domäne, Prolyl-Hydroxylase 2 (PHD2), humanem β-Defensin-2 (hBD2), humaner Interleukin-6-Rezeptor-α-Kette (hIL-6Rα), Mannan bindendem Lectin-CLR (MBL-CLR), peroxisomaler Ascorbatperoxidase (pAPX) des Weizens, Rhesusaffen-Interleukin-4 (rMamu IL-4) und α-Galactosidase gewählt ist.

8. Verfahren nach Anspruch 6, welches des Weiteren den Schritt des Isolierens und Reinigens des Thioredoxin-Fusionsproteins vor Schritt (iii) umfasst.

## Revendications

1. Plasmide qui est préparé par suppression de la séquence codant pour l'étiquette S à partir du plasmide pET-32a(+) ayant la séquence nucléotidique de SEQ ID NO : 3 représentée par la carte de restriction dans la Fig. 1, le plasmide pET-32a(+) comprenant un gène de thiorédoxin, un site de reconnaissance de thrombine, une séquence codant pour l'étiquette S, et un site de clonage multiple pour l'insertion d'un gène codant pour une protéine cible, le plasmide ayant la séquence nucléotidique de SEQ ID NO : 4 représentée par la carte de restriction dans la Fig. 2, et étant dénommé plasmide pET-32-S(-).

2. Plasmide selon la revendication 1, le gène codant pour une protéine cible étant inséré dans le site de clonage multiple du plasmide pET-32-S(-).

3. Plasmide selon la revendication 2, ladite protéine cible étant choisie dans le groupe comprenant le STAT6 SH2 domaine humain, la prolyl hydroxylase 2 (PHD2), la β-défensine-2 humaine (hBD2), la chaîne α du récepteur interleukine-6 humain (hIL-6Rα), le CLR de lectine liant de mannan (MBL-CLR), la peroxidase ascorbate des peroxisomes du blé (pAPX), l'interleukine-4 du macaque rhésus (rMamu IL-4), et la galactosidase α.

4. Cellule d'*E*. *coli,* transformée par le plasmide selon l'une quelconque des revendications 1 et 2.

5. Cellule d'*E*. *coli* selon la revendication 4, qui est choisie dans le groupe comprenant de DH5α, BL21, HB101, JM109 et TH2.

6. Procédé de production d'une protéine cible, comprenant :
i) l'insertion d'un gène codant pour le protéine cible dans le site de clonage multiple du plasmide selon la revendication 1 ;
ii) la transformation de cellules d'*E*. *coli* par le plasmide obtenu dans i), et la culture des cellules d'*E*. *coli* transformées afin d'exprimer une protéine de fusion de thiorédoxine ; et
iii) le traitement de la protéine de fusion de thiorédoxine avec de la thrombine.

7. Procédé selon la revendication 6, la protéine cible étant choisie dans la groupe comprenant le STAT6 SH2 domaine humain, la prolyl hydroxylase 2 (PHD2), la β-défensine-2 humaine (hBD2), la chaîne α du récepteur interleukine-6 humain (hIL-6Rα), le CLR de lectine liant de mannan (MBL-CLR), la peroxidase ascorbate des peroxisomes du blé (pAPX), l'interleukine-4 du macaque rhésus (rMamu IL-4), et la galactosidase α

8. Procédé selon la revendication 6, comprenant en outre des étapes d'isolation et de purification de la protéine de fusion de thiorédoxine préalablement à l'étape (iii).


<110> AHN, Hee-chul
   AHN, Hyung Jun
   SHIN, Dong Yun
   LIM, Jong Soo
   AHN, Dae-Ro
<120> PLASMID FOR EXPRESSION OF THIOREDOXIN-FUSED PROTEIN AND METHOD OF PRODUCING A TARGET PROTEIN USING SAME
<130> EP66637HV163
<140> not yet assigned
   <141> herewith
<150> KR 10-2009-0003431
   <151> 2009-01-15
<160> 7
<170> KopatentIn 1.71
<210> 1
   <211> 48
   <212> DNA
   <213> Artificial Sequence
<220>
   <223> Primer for site-specific deletion mutagenesis of pET-32a(+)
<400> 1
   tctggtctgg tgccacgcgg ttctgccatg gctgatatcg gatccgaa 48
<210> 2
   <211> 48
   <212> DNA
   <213> Artificial Sequence
<220>
   <223> Primer for site-specific deletion mutagenesis of pET-32a(+)
<400> 2
   ttcggatccg atatcagcca tggcagaacc gcgtggcacc agaccaga 48
<210> 3
   <211> 5900
   <212> DNA
   <213> Artificial Sequence
<220>
   <223> synthetic pET-32a(+) plasmid
<400> 3
   atccggatat agttcctcct ttcagcaaaa aacccctcaa gacccgttta gaggccccaa 60
   ggggttatgc tagttattgc tcagcggtgg cagcagccaa ctcagcttcc tttcgggctt 120
   tgttagcagc cggatctcag tggtggtggt ggtggtgctc gagtgcggcc gcaagcttgt 180
   cgacggagct cgaattcgga tccgatatca gccatggcct tgtcgtcgtc gtcggtaccc 240
   agatctgggc tgtccatgtg ctggcgttcg aatttagcag cagcggtttc tttcatacca 300
   gaaccgcgtg gcaccagacc agaagaatga tgatgatgat ggtgcatatg gccagaacca 360
   gaaccggcca ggttagcgtc gaggaactct ttcaactgac ctttagacag tgcacccact        420
   ttggttgccg ccacttcacc gtttttgaac agcagcagag tcgggatacc acggatgcca        480
   tatttcggcg cagtgccagg gttttgatcg atgttcagtt ttgcaacggt cagtttgccc        540
   tgatattcgt cagcgatttc atccagaatc ggggcgatca ttttgcacgg accgcaccac        600
   tctgcccaga aatcgacgag gatcgccccg tccgctttga gtacatccgt gtcaaaactg        660
   tcgtcagtca ggtgaataat tttatcgctc atatgtatat ctccttctta aagttaaaca        720
   aaattatttc tagaggggaa ttgttatccg ctcacaattc ccctatagtg agtcgtatta        780
   atttcgcggg atcgagatcg atctcgatcc tctacgccgg acgcatcgtg gccggcatca        840
   ccggcgccac aggtgcggtt gctggcgcct atatcgccga catcaccgat ggggaagatc        900
   gggctcgcca cttcgggctc atgagcgctt gtttcggcgt gggtatggtg gcaggccccg        960
   tggccggggg actgttgggc gccatctcct tgcatgcacc attccttgcg gcggcggtgc       1020
   tcaacggcct caacctacta ctgggctgct tcctaatgca ggagtcgcat aagggagagc       1080
   gtcgagatcc cggacaccat cgaatggcgc aaaacctttc gcggtatggc atgatagcgc       1140
   ccggaagaga gtcaattcag ggtggtgaat gtgaaaccag taacgttata cgatgtcgca       1200
   gagtatgccg gtgtctctta tcagaccgtt tcccgcgtgg tgaaccaggc cagccacgtt       1260
   tctgcgaaaa cgcgggaaaa agtggaagcg gcgatggcgg agctgaatta cattcccaac       1320
   cgcgtggcac aacaactggc gggcaaacag tcgttgctga ttggcgttgc cacctccagt       1380
   ctggccctgc acgcgccgtc gcaaattgtc gcggcgatta aatctcgcgc cgatcaactg       1440
   ggtgccagcg tggtggtgtc gatggtagaa cgaagcggcg tcgaagcctg taaagcggcg       1500
   gtgcacaatc ttctcgcgca acgcgtcagt gggctgatca ttaactatcc gctggatgac       1560
   caggatgcca ttgctgtgga agctgcctgc actaatgttc cggcgttatt tcttgatgtc       1620
   tctgaccaga cacccatcaa cagtattatt ttctcccatg aagacggtac gcgactgggc       1680
   gtggagcatc tggtcgcatt gggtcaccag caaatcgcgc tgttagcggg cccattaagt       1740
   tctgtctcgg cgcgtctgcg tctggctggc tggcataaat atctcactcg caatcaaatt       1800
   cagccgatag cggaacggga aggcgactgg agtgccatgt ccggttttca acaaaccatg       1860
   caaatgctga atgagggcat cgttcccact gcgatgctgg ttgccaacga tcagatggcg       1920
   ctgggcgcaa tgcgcgccat taccgagtcc gggctgcgcg ttggtgcgga catctcggta       1980
   gtgggatacg acgataccga agacagctca tgttatatcc cgccgttaac caccatcaaa       2040
   caggattttc gcctgctggg gcaaaccagc gtggaccgct tgctgcaact ctctcagggc       2100
   caggcggtga agggcaatca gctgttgccc gtctcactgg tgaaaagaaa aaccaccctg       2160
   gcgcccaata cgcaaaccgc ctctccccgc gcgttggccg attcattaat gcagctggca       2220
   cgacaggttt cccgactgga aagcgggcag tgagcgcaac gcaattaatg taagttagct       2280
   cactcattag gcaccgggat ctcgaccgat gcccttgaga gccttcaacc cagtcagctc       2340
   cttccggtgg gcgcggggca tgactatcgt cgccgcactt atgactgtct tctttatcat       2400
   gcaactcgta ggacaggtgc cggcagcgct ctgggtcatt ttcggcgagg accgctttcg 2460
   ctggagcgcg acgatgatcg gcctgtcgct tgcggtattc ggaatcttgc acgccctcgc 2520
   tcaagccttc gtcactggtc ccgccaccaa acgtttcggc gagaagcagg ccattatcgc 2580
   cggcatggcg gccccacggg tgcgcatgat cgtgctcctg tcgttgagga cccggctagg 2640
   ctggcggggt tgccttactg gttagcagaa tgaatcaccg atacgcgagc gaacgtgaag 2700
   cgactgctgc tgcaaaacgt ctgcgacctg agcaacaaca tgaatggtct tcggtttccg 2760
   tgtttcgtaa agtctggaaa cgcggaagtc agcgccctgc accattatgt tccggatctg 2820
   catcgcagga tgctgctggc taccctgtgg aacacctaca tctgtattaa cgaagcgctg 2880
   gcattgaccc tgagtgattt ttctctggtc ccgccgcatc cataccgcca gttgtttacc 2940
   ctcacaacgt tccagtaacc gggcatgttc atcatcagta acccgtatcg tgagcatcct 3000
   ctctcgtttc atcggtatca ttacccccat gaacagaaat cccccttaca cggaggcatc 3060
   agtgaccaaa caggaaaaaa ccgcccttaa catggcccgc tttatcagaa gccagacatt 3120
   aacgcttctg gagaaactca acgagctgga cgcggatgaa caggcagaca tctgtgaatc 3180
   gcttcacgac cacgctgatg agctttaccg cagctgcctc gcgcgtttcg gtgatgacgg 3240
   tgaaaacctc tgacacatgc agctcccgga gacggtcaca gcttgtctgt aagcggatgc 3300
   cgggagcaga caagcccgtc agggcgcgtc agcgggtgtt ggcgggtgtc ggggcgcagc 3360
   catgacccag tcacgtagcg atagcggagt gtatactggc ttaactatgc ggcatcagag 3420
   cagattgtac tgagagtgca ccatatatgc ggtgtgaaat accgcacaga tgcgtaagga 3480
   gaaaataccg catcaggcgc tcttccgctt cctcgctcac tgactcgctg cgctcggtcg 3540
   ttcggctgcg gcgagcggta tcagctcact caaaggcggt aatacggtta tccacagaat 3600
   caggggataa cgcaggaaag aacatgtgag caaaaggcca gcaaaaggcc aggaaccgta 3660
   aaaaggccgc gttgctggcg tttttccata ggctccgccc ccctgacgag catcacaaaa 3720
   atcgacgctc aagtcagagg tggcgaaacc cgacaggact ataaagatac caggcgtttc 3780
   cccctggaag ctccctcgtg cgctctcctg ttccgaccct gccgcttacc ggatacctgt 3840
   ccgcctttct cccttcggga agcgtggcgc tttctcatag ctcacgctgt aggtatctca 3900
   gttcggtgta ggtcgttcgc tccaagctgg gctgtgtgca cgaacccccc gttcagcccg 3960
   accgctgcgc cttatccggt aactatcgtc ttgagtccaa cccggtaaga cacgacttat 4020
   cgccactggc agcagccact ggtaacagga ttagcagagc gaggtatgta ggcggtgcta 4080
   cagagttctt gaagtggtgg cctaactacg gctacactag aaggacagta tttggtatct 4140
   gcgctctgct gaagccagtt accttcggaa aaagagttgg tagctcttga tccggcaaac 4200
   aaaccaccgc tggtagcggt ggtttttttg tttgcaagca gcagattacg cgcagaaaaa 4260
   aaggatctca agaagatcct ttgatctttt ctacggggtc tgacgctcag tggaacgaaa 4320
   actcacgtta agggattttg gtcatgagat tatcaaaaag gatcttcacc tagatccttt 4380
   taaattaaaa atgaagtttt aaatcaatct aaagtatata tgagtaaact tggtctgaca 4440
   gttaccaatg cttaatcagt gaggcaccta tctcagcgat ctgtctattt cgttcatcca 4500
   tagttgcctg actccccgtc gtgtagataa ctacgatacg ggagggctta ccatctggcc 4560
   ccagtgctgc aatgataccg cgagacccac gctcaccggc tccagattta tcagcaataa 4620
   accagccagc cggaagggcc gagcgcagaa gtggtcctgc aactttatcc gcctccatcc 4680
   agtctattaa ttgttgccgg gaagctagag taagtagttc gccagttaat agtttgcgca 4740
   acgttgttgc cattgctgca ggcatcgtgg tgtcacgctc gtcgtttggt atggcttcat 4800
   tcagctccgg ttcccaacga tcaaggcgag ttacatgatc ccccatgttg tgcaaaaaag 4860
   cggttagctc cttcggtcct ccgatcgttg tcagaagtaa gttggccgca gtgttatcac 4920
   tcatggttat ggcagcactg cataattctc ttactgtcat gccatccgta agatgctttt 4980
   ctgtgactgg tgagtactca accaagtcat tctgagaata gtgtatgcgg cgaccgagtt 5040
   gctcttgccc ggcgtcaata cgggataata ccgcgccaca tagcagaact ttaaaagtgc 5100
   tcatcattgg aaaacgttct tcggggcgaa aactctcaag gatcttaccg ctgttgagat 5160
   ccagttcgat gtaacccact cgtgcaccca actgatcttc agcatctttt actttcacca 5220
   gcgtttctgg gtgagcaaaa acaggaaggc aaaatgccgc aaaaaaggga ataagggcga 5280
   cacggaaatg ttgaatactc atactcttcc tttttcaata ttattgaagc atttatcagg 5340
   gttattgtct catgagcgga tacatatttg aatgtattta gaaaaataaa caaatagggg 5400
   ttccgcgcac atttccccga aaagtgccac ctgaaattgt aaacgttaat attttgttaa 5460
   aattcgcgtt aaatttttgt taaatcagct cattttttaa ccaataggcc gaaatcggca 5520
   aaatccctta taaatcaaaa gaatagaccg agatagggtt gagtgttgtt ccagtttgga 5580
   acaagagtcc actattaaag aacgtggact ccaacgtcaa agggcgaaaa accgtctatc 5640
   agggcgatgg cccactacgt gaaccatcac cctaatcaag ttttttgggg tcgaggtgcc 5700
   gtaaagcact aaatcggaac cctaaaggga gcccccgatt tagagcttga cggggaaagc 5760
   cggcgaacgt ggcgagaaag gaagggaaga aagcgaaagg agcgggcgct agggcgctgg 5820
   caagtgtagc ggtcacgctg cgcgtaacca ccacacccgc cgcgcttaat gcgccgctac 5880
   agggcgcgtc ccattcgcca 5900
<210> 4
   <211> 5819
   <212> DNA
   <213> Artificial Sequence
<220>
   <223> Synthetic pET-32-S(-) plasmid
<400> 4
   atccggatat agttcctcct ttcagcaaaa aacccctcaa gacccgttta gaggccccaa 60
   ggggttatgc tagttattgc tcagcggtgg cagcagccaa ctcagcttcc tttcgggctt 120
   tgttagcagc cggatctcag tggtggtggt ggtggtgctc gagtgcggcc gcaagcttgt 180
   cgacggagct cgaattcgga tccgatatca gccatggcag aaccgcgtgg caccagacca 240
   gaagaatgat gatgatgatg gtgcatatgg ccagaaccag aaccggccag gttagcgtcg 300
   aggaactctt tcaactgacc tttagacagt gcacccactt tggttgccgc cacttcaccg 360
   tttttgaaca gcagcagagt cgggatacca cggatgccat atttcggcgc agtgccaggg 420
   ttttgatcga tgttcagttt tgcaacggtc agtttgccct gatattcgtc agcgatttca 480
   tccagaatcg ggcgatcatt tttgcacgga ccgcaccact ctgcccagaa atcgacgagg 540
   atcgccccgt ccgctttgag tacatccgtg tcaaaactgt cgtcagtcag gtgaataatt 600
   ttatcgctca tatgtatatc tccttcttaa agttaaacaa aattatttct agaggggaat 660
   tgttatccgc tcacaattcc cctatagtga gtcgtattaa tttcgcggga tcgagatcga 720
   tctcgatcct ctacgccgga cgcatcgtgg ccggcatcac cggcgccaca ggtgcggttg 780
   ctggcgccta tatcgccgac atcaccgatg gggaagatcg ggctcgccac ttcgggctca 840
   tgagcgcttg tttcggcgtg ggtatggtgg caggccccgt ggccggggga ctgttgggcg 900
   ccatctcctt gcatgcacca ttccttgcgg cggcggtgct caacggcctc aacctactac 960
   tgggctgctt cctaatgcag gagtcgcata agggagagcg tcgagatccc ggacaccatc 1020
   gaatggcgca aaacctttcg cggtatggca tgatagcgcc cggaagagag tcaattcagg 1080
   gtggtgaatg tgaaaccagt aacgttatac gatgtcgcag agtatgccgg tgtctcttat 1140
   cagaccgttt cccgcgtggt gaaccaggcc agccacgttt ctgcgaaaac gcgggaaaaa 1200
   gtggaagcgg cgatggcgga gctgaattac attcccaacc gcgtggcaca acaactggcg 1260
   ggcaaacagt cgttgctgat tggcgttgcc acctccagtc tggccctgca cgcgccgtcg 1320
   caaattgtcg cggcgattaa atctcgcgcc gatcaactgg gtgccagcgt ggtggtgtcg 1380
   atggtagaac gaagcggcgt cgaagcctgt aaagcggcgg tgcacaatct tctcgcgcaa 1440
   cgcgtcagtg ggctgatcat taactatccg ctggatgacc aggatgccat tgctgtggaa 1500
   gctgcctgca ctaatgttcc ggcgttattt cttgatgtct ctgaccagac acccatcaac 1560
   agtattattt tctcccatga agacggtacg cgactgggcg tggagcatct ggtcgcattg 1620
   ggtcaccagc aaatcgcgct gttagcgggc ccattaagtt ctgtctcggc gcgtctgcgt 1680
   ctggctggct ggcataaata tctcactcgc aatcaaattc agccgatagc ggaacgggaa 1740
   ggcgactgga gtgccatgtc cggttttcaa caaaccatgc aaatgctgaa tgagggcatc 1800
   gttcccactg cgatgctggt tgccaacgat cagatggcgc tgggcgcaat gcgcgccatt 1860
   accgagtccg ggctgcgcgt tggtgcggac atctcggtag tgggatacga cgataccgaa 1920
   gacagctcat gttatatccc gccgttaacc accatcaaac aggattttcg cctgctgggg 1980
   caaaccagcg tggaccgctt gctgcaactc tctcagggcc aggcggtgaa gggcaatcag 2040
   ctgttgcccg tctcactggt gaaaagaaaa accaccctgg cgcccaatac gcaaaccgcc 2100
   tctccccgcg cgttggccga ttcattaatg cagctggcac gacaggtttc ccgactggaa 2160
   agcgggcagt gagcgcaacg caattaatgt aagttagctc actcattagg caccgggatc 2220
   tcgaccgatg cccttgagag ccttcaaccc agtcagctcc ttccggtggg cgcggggcat 2280
   gactatcgtc gccgcactta tgactgtctt ctttatcatg caactcgtag gacaggtgcc 2340
   ggcagcgctc tgggtcattt tcggcgagga ccgctttcgc tggagcgcga cgatgatcgg 2400
   cctgtcgctt gcggtattcg gaatcttgca cgccctcgct caagccttcg tcactggtcc 2460
   cgccaccaaa cgtttcggcg agaagcaggc cattatcgcc ggcatggcgg ccccacgggt 2520
   gcgcatgatc gtgctcctgt cgttgaggac ccggctaggc tggcggggtt gccttactgg 2580
   ttagcagaat gaatcaccga tacgcgagcg aacgtgaagc gactgctgct gcaaaacgtc 2640
   tgcgacctga gcaacaacat gaatggtctt cggtttccgt gtttcgtaaa gtctggaaac 2700
   gcggaagtca gcgccctgca ccattatgtt ccggatctgc atcgcaggat gctgctggct 2760
   accctgtgga acacctacat ctgtattaac gaagcgctgg cattgaccct gagtgatttt 2820
   tctctggtcc cgccgcatcc ataccgccag ttgtttaccc tcacaacgtt ccagtaaccg 2880
   ggcatgttca tcatcagtaa cccgtatcgt gagcatcctc tctcgtttca tcggtatcat 2940
   tacccccatg aacagaaatc ccccttacac ggaggcatca gtgaccaaac aggaaaaaac 3000
   cgcccttaac atggcccgct ttatcagaag ccagacatta acgcttctgg agaaactcaa 3060
   cgagctggac gcggatgaac aggcagacat ctgtgaatcg cttcacgacc acgctgatga 3120
   gctttaccgc agctgcctcg cgcgtttcgg tgatgacggt gaaaacctct gacacatgca 3180
   gctcccggag acggtcacag cttgtctgta agcggatgcc gggagcagac aagcccgtca 3240
   gggcgcgtca gcgggtgttg gcgggtgtcg gggcgcagcc atgacccagt cacgtagcga 3300
   tagcggagtg tatactggct taactatgcg gcatcagagc agattgtact gagagtgcac 3360
   catatatgcg gtgtgaaata ccgcacagat gcgtaaggag aaaataccgc atcaggcgct 3420
   cttccgcttc ctcgctcact gactcgctgc gctcggtcgt tcggctgcgg cgagcggtat 3480
   cagctcactc aaaggcggta atacggttat ccacagaatc aggggataac gcaggaaaga 3540
   acatgtgagc aaaaggccag caaaaggcca ggaaccgtaa aaaggccgcg ttgctggcgt 3600
   ttttccatag gctccgcccc cctgacgagc atcacaaaaa tcgacgctca agtcagaggt 3660
   ggcgaaaccc gacaggacta taaagatacc aggcgtttcc ccctggaagc tccctcgtgc 3720
   gctctcctgt tccgaccctg ccgcttaccg gatacctgtc cgcctttctc ccttcgggaa 3780
   gcgtggcgct ttctcatagc tcacgctgta ggtatctcag ttcggtgtag gtcgttcgct 3840
   ccaagctggg ctgtgtgcac gaaccccccg ttcagcccga ccgctgcgcc ttatccggta 3900
   actatcgtct tgagtccaac ccggtaagac acgacttatc gccactggca gcagccactg 3960
   gtaacaggat tagcagagcg aggtatgtag gcggtgctac agagttcttg aagtggtggc 4020
   ctaactacgg ctacactaga aggacagtat ttggtatctg cgctctgctg aagccagtta 4080
   ccttcggaaa aagagttggt agctcttgat ccggcaaaca aaccaccgct ggtagcggtg 4140
   gtttttttgt ttgcaagcag cagattacgc gcagaaaaaa aggatctcaa gaagatcctt 4200
   tgatcttttc tacggggtct gacgctcagt ggaacgaaaa ctcacgttaa gggattttgg 4260
   tcatgagatt atcaaaaagg atcttcacct agatcctttt aaattaaaaa tgaagtttta 4320
   aatcaatcta aagtatatat gagtaaactt ggtctgacag ttaccaatgc ttaatcagtg 4380
   aggcacctat ctcagcgatc tgtctatttc gttcatccat agttgcctga ctccccgtcg 4440
   tgtagataac tacgatacgg gagggcttac catctggccc cagtgctgca atgataccgc 4500
   gagacccacg ctcaccggct ccagatttat cagcaataaa ccagccagcc ggaagggccg 4560
   agcgcagaag tggtcctgca actttatccg cctccatcca gtctattaat tgttgccggg 4620
   aagctagagt aagtagttcg ccagttaata gtttgcgcaa cgttgttgcc attgctgcag 4680
   gcatcgtggt gtcacgctcg tcgtttggta tggcttcatt cagctccggt tcccaacgat 4740
   caaggcgagt tacatgatcc cccatgttgt gcaaaaaagc ggttagctcc ttcggtcctc 4800
   cgatcgttgt cagaagtaag ttggccgcag tgttatcact catggttatg gcagcactgc 4860
   ataattctct tactgtcatg ccatccgtaa gatgcttttc tgtgactggt gagtactcaa 4920
   ccaagtcatt ctgagaatag tgtatgcggc gaccgagttg ctcttgcccg gcgtcaatac 4980
   gggataatac cgcgccacat agcagaactt taaaagtgct catcattgga aaacgttctt 5040
   cggggcgaaa actctcaagg atcttaccgc tgttgagatc cagttcgatg taacccactc 5100
   gtgcacccaa ctgatcttca gcatctttta ctttcaccag cgtttctggg tgagcaaaaa 5160
   caggaaggca aaatgccgca aaaaagggaa taagggcgac acggaaatgt tgaatactca 5220
   tactcttcct ttttcaatat tattgaagca tttatcaggg ttattgtctc atgagcggat 5280
   acatatttga atgtatttag aaaaataaac aaataggggt tccgcgcaca tttccccgaa 5340
   aagtgccacc tgaaattgta aacgttaata ttttgttaaa attcgcgtta aatttttgtt 5400
   aaatcagctc attttttaac caataggccg aaatcggcaa aatcccttat aaatcaaaag 5460
   aatagaccga gatagggttg agtgttgttc cagtttggaa caagagtcca ctattaaaga 5520
   acgtggactc caacgtcaaa gggcgaaaaa ccgtctatca gggcgatggc ccactacgtg 5580
   aaccatcacc ctaatcaagt tttttggggt cgaggtgccg taaagcacta aatcggaacc 5640
   ctaaagggag cccccgattt agagcttgac ggggaaagcc ggcgaacgtg gcgagaaagg 5700
   aagggaagaa agcgaaagga gcgggcgcta gggcgctggc aagtgtagcg gtcacgctgc 5760
   gcgtaaccac cacacccgcc gcgcttaatg cgccgctaca gggcgcgtcc cattcgcca 5819
<210> 5
   <211> 2544
   <212> DNA
   <213> Artificial Sequence
<220>
   <223> Human STAT6 cDNA
<400> 5
   atgtctctgt ggggtctggt ctccaagatg cccccagaaa aagtgcagcg gctctatgtc        60
   gactttcccc aacacctgcg gcatcttctg ggtgactggc tggagagcca gccctgggag       120
   ttcctggtcg gctccgacgc cttctgctgc aacttggcta gtgccctact ttcagacact       180
   gtccagcacc ttcaggcctc ggtgggagag cagggggagg ggagcaccat cttgcaacac    240
   atcagcaccc ttgagagcat atatcagagg gaccccctga agctggtggc cactttcaga    300
   caaatacttc aaggagagaa aaaagctgtt atggaacagt tccgccactt gccaatgcct    360
   ttccactgga agcaggaaga actcaagttt aagacaggct tgcggaggct gcagcaccga    420
   gtaggggaga tccaccttct ccgagaagcc ctgcagaagg gggctgaggc tggccaagtg    480
   tctctgcaca gcttgataga aactcctgct aatgggactg ggccaagtga ggccctggcc    540
   atgctactgc aggagaccac tggagagcta gaggcagcca aagccctagt gctgaagagg    600
   atccagattt ggaaacggca gcagcagctg gcagggaatg gcgcaccgtt tgaggagagc    660
   ctggccccac tccaggagag gtgtgaaagc ctggtggaca tttattccca gctacagcag    720
   gaggtagggg cggctggtgg ggagcttgag cccaagaccc gggcatcgct gactggccgg    780
   ctggatgaag tcctgagaac cctcgtcacc agttgcttcc tggtggagaa gcagcccccc    840
   caggtactga agactcagac caagttccag gctggagttc gattcctgtt gggcttgagg    900
   ttcctggggg ccccagccaa gcctccgctg gtcagggccg acatggtgac agagaagcag    960
   gcgcgggagc tgagtgtgcc tcagggtcct ggggctggag cagaaagcac tggagaaatc   1020
   atcaacaaca ctgtgccctt ggagaacagc attcctggga actgctgctc tgccctgttc   1080
   aagaacctgc ttctcaagaa gatcaagcgg tgtgagcgga agggcactga gtctgtcaca   1140
   gaggagaagt gcgctgtgct cttctctgcc agcttcacac ttggccccgg caaactcccc   1200
   atccagctcc aggccctgtc tctgcccctg gtggtcatcg tccatggcaa ccaagacaac   1260
   aatgccaaag ccactatcct gtgggacaat gccttctctg agatggaccg cgtgcccttt   1320
   gtggtggctg agcgggtgcc ctgggagaag atgtgtgaaa ctctgaacct gaagttcatg   1380
   gctgaggtgg ggaccaaccg ggggctgctc ccagagcact tcctcttcct ggcccagaag   1440
   atcttcaatg acaacagcct cagtatggag gccttccagc accgttctgt gtcctggtcg   1500
   cagttcaaca aggagatcct gctgggccgt ggcttcacct tttggcagtg gtttgatggt   1560
   gtcctggacc tcaccaaacg ctgtctccgg agctactggt ctgaccggct gatcattggc   1620
   ttcatcagca aacagtacgt tactagcctt cttctcaatg agcccgacgg aacctttctc   1680
   ctccgcttca gcgactcaga gattgggggc atcaccattg cccatgtcat ccggggccag   1740
   gatggctctc cacagataga gaacatccag ccattctctg ccaaagacct gtccattcgc   1800
   tcactggggg accgaatccg ggatcttgct cagctcaaaa atctctatcc caagaagccc   1860
   aaggatgagg ctttccggag ccactacaag cctgaacaga tgggtaagga tggcaggggt   1920
   tatgtcccag ctaccatcaa gatgaccgtg gaaagggacc aaccacttcc taccccagag   1980
   ctccagatgc ctaccatggt gccttcttat gaccttggaa tggcccctga ttcctccatg   2040
   agcatgcagc ttggcccaga tatggtgccc caggtgtacc caccacactc tcactccatc   2100
   cccccgtatc aaggcctctc cccagaagaa tcagtcaacg tgttgtcagc cttccaggag   2160
   cctcacctgc agatgccccc cagcctgggc cagatgagcc tgccctttga ccagcctcac   2220
   ccccagggcc tgctgccgtg ccagcctcag gagcatgctg tgtccagccc tgaccccctg        2280
   ctctgctcag atgtgaccat ggtggaagac agctgcctga gccagccagt gacagcgttt        2340
   cctcagggca cttggattgg tgaagacata ttccctcctc tgctgcctcc cactgaacag        2400
   gacctcacta agcttctcct ggaggggcaa ggggagtcgg ggggagggtc cttgggggca        2460
   cagcccctcc tgcagccctc ccactatggg caatctggga tctcaatgtc ccacatggac        2520
   ctaagggcca accccagttg gtga                                               2544
<210> 6
   <211> 35
   <212> DNA
   <213> Artificial Sequence
<220>
   <223> Primer for amplification of STAT6 cDNA
<400> 6
   aaactcgagt cagagctctg gggtaggaag tggtt 35
<210> 7
   <211> 32
   <212> DNA
   <213> Artificial Sequence
<220>
   <223> Primer for amplification of STAT6 cDNA
<400> 7
   aaagaattcg acctcaccaa acgctgtctc cg 32